# EUROPEAN PATENT SPECIFICATION

(11) **EP 3 397 066 B1**
(45) Date of publication and mention of the grant of the patent: **16.09.2020**
(21) Application number: 16868083.3
(22) Date of filing: 24.11.2016
(51) Int. Cl.: A23L 33/17, A23L 33/185, A23J 1/00, A23J 1/12, A23J 1/14, B01D 11/02, C07K 1/36, A23K 10/38, A23K 10/20, A23K 20/147

(54) **PROCESS FOR SEPARATING PROTEINS FROM BIOMASS MATERIALS**
VERFAHREN ZUR TRENNUNG VON PROTEINEN AUS BIOMASSEMATERIALIEN
PROCÉDÉ DE SÉPARATION DE PROTÉINES À PARTIR DE MATIÈRES DE BIOMASSE

(30) Priority: 27.11.2015 FI 20155889
(43) Date of publication of application: 07.11.2018
(73) Proprietor: Teknologian Tutkimuskeskus VTT OY, 02150 Espoo (FI)
(72) Inventor: WAHLSTRÖM, Ronny, 02044 VTT (FI); KUUTTI, Lauri, 02044 VTT (FI); HILTUNEN, Jaakko, 02044 VTT (FI); VUOTI, Sauli, 02044 VTT (FI); ERCILI-CURA, Dilek, 02044 VTT (FI); ROMMI, Katariina, 02044 VTT (FI)
(74) Representative: Boco IP Oy Ab
(86) International application number: PCT/FI2016/050827
(87) International publication number: WO 2017/089655

(56) References cited:
- EP-A2- 0 289 183
- WO-A1-03/013267
- WO-A1-2011/155829
- WO-A1-2013/153203
- CA-C- 1 317 413
- CN-A- 1 314 089
- US-A- 3 970 614
- US-A1- 2006 241 287
- QUN ZENG ET AL: "Deep eutectic solvents as novel extraction media for protein partitioning", ANALYST, vol. 139, no. 10, 1 January 2014 (2014-01-01), page 2565, XP055599635, UK ISSN: 0003-2654, DOI: 10.1039/c3an02235h
- YUNTAO DAI ET AL: "Ionic Liquids and Deep Eutectic Solvents in Natural Products Research: Mixtures of Solids as Extraction Solvents", JOURNAL OF NATURAL PRODUCTS., vol. 76, no. 11, 22 November 2013 (2013-11-22), pages 2162-2173, XP055236247, US ISSN: 0163-3864, DOI: 10.1021/np400051w
- XU K. ET AL.: 'A green deep eutectic solvent-based aqueous two-phase system for protein extracting.' ANALYTICA CHIMICA ACTA vol. 864, 2015, pages 9 - 20, XP055385138
- PARNICA J. ET AL.: 'Urea and guanidine salts as novel components for deep eutectic solvents.' JOURNAL OF MOLECULAR LIQUIDS vol. 197, 2014, pages 23 - 26, XP029034958

## Description

### FIELD OF THE INVENTION

The present invention relates to a process for separating proteins from biomass materials. More particularly the invention relates to a process, where biomass material comprising proteins is treated with a deep eutectic solvent (DES) for providing proteins and protein fractions with high yields and purity. The disclosure relates further to the use of the obtained proteins and protein fractions in foods and in animal feeds.

### BACKGROUND OF THE INVENTION

There is constantly an outstanding protein shortage in several parts of the world, and further, a major part of the current protein production is not carried out in a sustainable way. The demand for protein products particularly in human nutrition and as animal feeds is enormous and continuously growing with the world population and rising living standards.

Many agricultural side-streams and waste materials, as well as side streams originating from the food industry contain high contents of protein. Said materials and side streams are currently utilized only to a limited extent. For value-added applications it is necessary to extract the proteins from the side stream matrix, but in many cases the currently used extraction methods with aqueous alkali solutions only provide low yields of extracted protein. The quality of the proteins may also be low after the alkali extraction, and typically unwanted compounds are co-extracted with the proteins necessitating further purification of the product.

In the alkali extraction method proteins are enriched from side streams of agrological origin with NaOH solutions at pH 10-12. Particularly, in the extraction of more recalcitrant starting materials, such as brewer's spent grain (BSG) or wheat bran (WB) the yields are not satisfactory. The high pH of the solution may lead to unwanted protein denaturation or even partial degradation. In general, one or several pH adjustment steps are needed in the work-up, leading to extensive use of acid and base chemicals as well as solvent water, which needs to be separated at a later point. Co-extraction of unwanted compounds is also a problem during the alkaline extraction of various feeds.

A eutectic mixture forms a eutectic with a melting point much lower than any of the individual components. The phenomenon was first described in 2003 for a mixture of choline chloride and urea in a 1:2 mole ratio, respectively. Choline chloride has a melting point of 302°C and that of urea is 133°C. The eutectic mixture melts at 12°C.

WO 2011/155829 A1 discloses a method for extracting materials from biological products using ionic liquids or deep eutectic solvents, particularly flavonoids, flavors and fragrances from plants, and antibiotics and colorants from microorganisms. US 2010/0163018 A1 relates to the fractionation of biomass materials with an ionic liquid to cellulose-enriched fractions and lignin-enriched fractions. Qun Zeng et al. 2014 [Analyst, 139 (10): 2565-2573, 2014] discloses deep eutectic solvents as novel extraction media for protein partitioning.

Despite the ongoing research and development of processing of agricultural side-streams and waste materials, as well as side streams originating from the food industry, there is still a need to provide an improved process for separating proteins from biomass materials.

### SUMMARY OF THE INVENTION

An object of the invention is to provide an improved process for separating proteins and/or protein fractions from biomass materials.

Another object of the invention is to provide a process for separating proteins and/or protein fractions from biomass materials, where biomass material comprising proteins is treated with a deep eutectic solvent (DES) for providing proteins and/or protein fractions with high yields and purity.

Disclosure relates to the use of the obtained proteins and protein fractions in foods and in animal feeds.

The present invention generally concerns a process for separating proteins and/or protein fractions from biomass materials, where biomass material comprising proteins is treated with a DES for providing proteins and/or protein fractions with high yields and purity.

Particularly, the invention relates to a process for separating proteins and/or protein fractions from biomass materials, where
in the first step biomass material containing streams and materials of plant or animal or algal or microbial origin, which contain proteins, or combinations thereof is mixed from 30 min to 30 hours at the temperature from 0°C to 100°C at the pressure 100 mbar - 30 bar with an aqueous deep eutectic solvent (DES) comprising a hydrogen bond acceptor selected from alkali metal carboxylates and earth alkaline metal carboxylates having the structure I: [X]^{m} Yₙ^{o}, where
X denotes an alkali metal or earth alkaline metal selected from Na, K, Li, Be, Mg or Ca, preferably Na or K;
Y denotes a mono- or polyvalent carboxylate, which may carry a straight alkane chain with a length between 1 and 20 carbons, an aromatic moiety, a branched alkane, a multivalent carboxylic acid, alcohol groups, alkenes and alkynes with chain lengths of 1 to 20 carbon units;
m denotes the charge of the alkali or earth alkaline metal ion, +1 or +2;
n denotes the number of carboxylate anions, 1 to 2; and
o denotes the overall charge of the carboxylate anion, -1 or -2,
and urea as hydrogen bond donor to obtain biomass mixture containing 1-40 wt% of the biomass material, calculated based on dry weight,
wherein deep eutectic solvent (DES) comprises a molar ratio of the alkali metal carboxylate or earth alkaline metal carboxylate to urea from 5:1 to 1:5 and the deep eutectic solvent (DES) comprises 0.01-4 mol of water with respect to the molar amount of the alkali metal carboxylate or earth alkaline metal carboxylate,
in the second step the biomass mixture is separated into a non-solubilized fraction and a liquid fraction, wherein 0 - 100°C water 1-8 times the volume of the deep eutectic solvent (DES) is added to the biomass mixture, the amount of water also containing the amounts of water possibly present in the first step or originating from the first step; and
in the third step, proteins are separated from the liquid fraction using technique based on molecular size selected from ultrafiltration, dialysis, membrane filtration methods and combinations thereof to yield a protein solution, or using technique based on isoelectric precipitation to yield a protein precipitate.

The disclosure also relates to the use of the obtained proteins and protein fractions in foods and in animal feeds.

Characteristic features of the invention are presented in the appended claims.

### DEFINITIONS

The term "alkali metal or earth alkaline metal carboxylate" means here a compound having the structure I, where X denotes an alkali metal or earth alkaline metal selected from Na, K, Li, Be, Mg or Ca, preferably Na or K and Y denotes a mono- or polyvalent carboxylate, which may carry a straight alkane chain with a length between 1 and 20 carbons, an aromatic moiety, a branched alkane, a multivalent carboxylic acid, alcohol groups, alkenes and alkynes with chain lengths of 1 to 20 carbon units.

Structure I: [X]^{m} Yₙ^{o}, in which m denotes the charge of the alkali or earth alkaline metal ion, +1 or +2, and n denotes the number of carboxylate anions, 1 to 2, and o denotes the overall charge of the carboxylate anion, -1 or -2.

### BRIEF DESCRIPTION OF DRAWINGS

Figure 1 illustrates an embodiment of the process.
Figure 2 illustrates the extraction efficiency of the process, where an embodiment using the DES NaAcO:Urea (molar ratio 1:2), containing 10 wt-% water, on brewer's spent grain at 3 extraction times is illustrated, and NaOH (pH 11) extraction is presented as reference.

### DETAILED DESCRIPTION OF THE INVENTION

It was surprisingly found that a high degree of protein extraction can be achieved, particularly on an industrial scale, by using the process of the invention, where biomass material comprising proteins is treated with a deep eutectic solvent (DES).

### Biomass material

Biomass material refers here to streams and materials of plant or animal or algal or microbial origin, which contain proteins. Preferably waste streams and side streams comprising plant material, animal material or algal material, particularly waste streams and side streams originating from agriculture and food industry are used. Examples of such biomass materials are side streams, such as press cakes from fruit industry, vine industry, beer industry and berry industry, and from edible oil pressing and refining industry, brewer's spent grain (BSG), rapeseed press cake (RSPC), wheat bran (WB), feathers, hair, hooves, claws, cartilage materials, and also beans and peas, which all contain high amounts of proteins.

The biomass material is suitably pretreated mechanically prior to introduction to the process of the invention.

Suitable mechanical pretreatment methods include grinding, milling, refining, crushing and cutting and any combinations thereof.

The biomass material may be dried prior to introduction to the process of the invention. The drying may be carried out using any drying methods known as such.

The biomass material may be sieved prior to using it as starting material in the process.

Suitably the average particle size of the biomass material entering the process is in the range of 0.05 - 10 mm, preferably 0.1 - 3 mm.

### Deep eutectic solvent useful in the process

The DES useful in the present process is selected from DESs comprising a hydrogen bond acceptor selected from alkali metal carboxylates and earth alkaline metal carboxylates, and urea as hydrogen bond donor.

The term "alkali metal or earth alkaline metal carboxylate" means here a compound having the structure I, where X denotes an alkali metal or earth alkaline metal selected from Na, K, Li, Be, Mg or Ca and Y denotes a mono- or polyvalent carboxylate, which may carry a straight alkane chain with a length between 1 and 20 carbons, an aromatic moiety, a branched alkane, a multivalent carboxylic acid, alcohol groups, alkenes and alkynes with chain lengths of 1 to 20 carbon units.

Structure I: [X]^{m} Yₙ^{o}, in which m denotes the charge of the alkali metal ion or earth alkaline metal ion, +1 or +2, and n denotes the number of carboxylate anions, 1 to 2, and o denotes the overall charge of the carboxylate anion, -1 or -2.
Preferably the alkali metal is selected from K or Na.

Suitably the carboxylate is a C1-C20 alkyl ester, preferably a C1-C5 alkyl ester, more preferably an acetate (AcO), formiate, oxalate or propionate, particularly preferably acetate.

Food grade DESs are preferred.

According to a preferable embodiment the DES is NaAcO:urea.

The DES comprises a molar ratio of the alkali metal carboxylate or earth alkaline metal carboxylate to urea from 5:1 to 1:5, preferably from 1:1 to 1:3, respectively.

According to an embodiment the DES comprises 0.01-4 mol of water with respect to the molar amount of the alkali metal carboxylate or earth alkaline metal carboxylate, preferably 0.5-1.5 mol of water.

NaAcO:urea is preferably used as an aqueous solution containing 28-37 wt% of NaAcO, 54-62 wt% of urea and 8-15 wt% of water.

The DES is suitably formed prior to introducing it to the process.

### Process

The process of the invention for separating proteins and/or protein fractions from biomass materials comprises the steps, where
in the first step biomass material containing streams and materials of plant or animal or algal or microbial origin, which contain proteins, or combinations thereof is mixed from 30 min to 30 hours at the temperature from 0°C to 100°C at the pressure 100 mbar - 30 bar with an aqueous deep eutectic solvent (DES) comprising a hydrogen bond acceptor selected from alkali metal carboxylates and earth alkaline metal carboxylates having the structure I: [X]^{m} Yₙ^{o}, where
X denotes an alkali metal or earth alkaline metal selected from Na, K, Li, Be, Mg or Ca, preferably Na or K;
Y denotes a mono- or polyvalent carboxylate, which may carry a straight alkane chain with a length between 1 and 20 carbons, an aromatic moiety, a branched alkane, a multivalent carboxylic acid, alcohol groups, alkenes and alkynes with chain lengths of 1 to 20 carbon units;
m denotes the charge of the alkali or earth alkaline metal ion, +1 or +2;
n denotes the number of carboxylate anions, 1 to 2; and
o denotes the overall charge of the carboxylate anion, -1 or -2,
and urea as hydrogen donor, to obtain biomass mixture containing 1-40 wt% of the biomass material, calculated based on dry weight,
wherein deep eutectic solvent (DES) comprises a molar ratio of the alkali metal carboxylate or earth alkaline metal carboxylate to urea from 5:1 to 1:5 and the deep eutectic solvent (DES) comprises 0.01-4 mol of water with respect to the molar amount of the alkali metal carboxylate or earth alkaline metal carboxylate,
in the second step the biomass mixture is separated into a non-solubilized fraction and a liquid fraction , wherein 0 - 100°C water 1-8 times the volume of the deep eutectic solvent (DES) is added to the biomass mixture, the amount of water also containing the amounts of water possibly present in the first step or originating from the first step; and
in the third step proteins are separated from the liquid fraction using technique based on molecular size selected from ultrafiltration, dialysis, membrane filtration methods and combinations thereof to yield a protein solution, or using technique based on isoelectric precipitation to yield a protein precipitate.

Preferably the biomass material is pretreated prior to introducing it to the process.

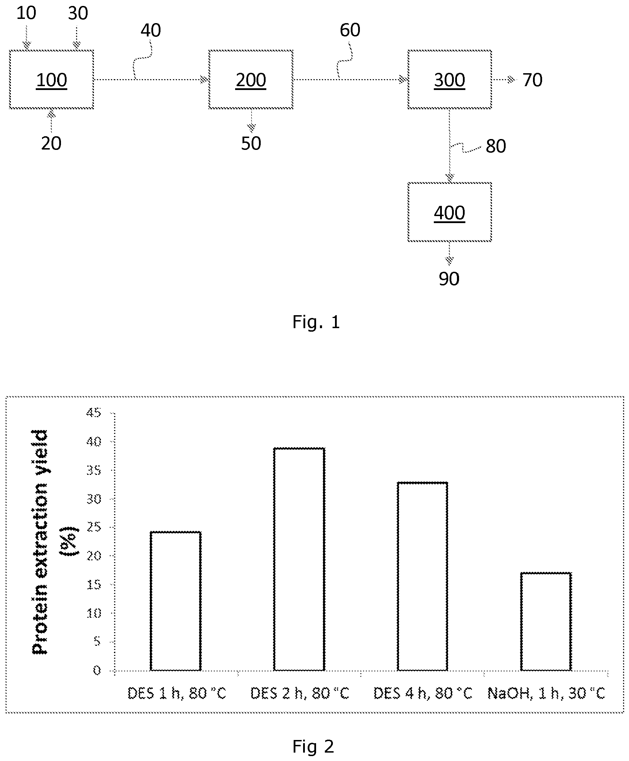

The biomass material may be dried prior to introducing it to the process. Drying methods known in the art may be used.
An embodiment of the invention is illustrated in Figure 1. Biomass material 10 and aqueous DES 20 are mixed in a vessel 100 to obtain a biomass mixture, water 30 is added to the biomass mixture and agitated, whereby an aqueous biomass mixture 40 is obtained. The aqueous biomass mixture 40 is separated into a non-solubilized fraction 50 and a liquid fraction 60 using a separator 200. The liquid fraction is separated in a separator 300 to a protein containing fraction 80 and non-protein residue 70. The protein containing fraction 80 is optionally dried in a drier (400) to yield dry protein product 90. In this embodiment water is added in the first step, however it is optional.

The separator 300 is a separator utilizing technique based on molecular size, or utilizing technique based on isoelectric precipitation.

### First step

In the first step biomass material is mixed with an aqueous DES comprising a hydrogen bond acceptor selected from alkali metal carboxylates and earth alkaline metal carboxylates, and urea as hydrogen bond donor to obtain biomass mixture.

The temperature in the first step is from 0°C to 100°C, preferably from 20°C to 80°C.

The mixing time in the first step is from 30 min to 30 hours, preferably from 1 to 16 hours, particularly preferably from 1.5 to 4 hours.

The biomass mixture may contain water not more than 20 wt%, preferably 10-20 wt%, calculated from the total weight of the biomass mixture.

Water may also be added to the biomass mixture or the biomass may contain the water. The amount of water in the biomass mixture may be decreased if necessary, for example by distillation, evaporation etc.

The DES comprises 0.01-4 mol of water with respect to the molar amount of the alkali metal carboxylate or earth alkaline metal carboxylate.

In the first step the biomass mixture contains 1-40 wt%, preferably 3-25 wt% of the biomass material, calculated based on dry weight.

In the first step the pressure is 100 mbar - 30 bar, preferably 1-20 bar. Inert gas, such as N₂, Ar, He or CO₂ may be used for pressurization of the reaction vessel.
Suitably any mixing devices may be used in the first step, such as high-shear mixers, homogenizers, processing grinders, helical mixers, high intensity mixers, fluidizers and twin-screw mixers may be used.

### Second step

In the second step the biomass mixture is separated into a non-solubilized fraction and a liquid fraction.

The biomass mixture is suitably mixed and after the mixing is completed, the biomass mixture is subjected to separation. Suitably the temperature of the biomass mixture in the separation is 40-100°C, preferably 60-85°C. The separation of the non-solubilized fraction from the liquid fraction is carried out suitably using sieves, centrifuges, filters, pressure filters etc., and suitably with the aid of pressure.

The non-solubilized fraction is suitably washed with water to remove all free proteins.

The non-solubilized fraction may be subjected to optional fibrillation.

The non-solubilized fraction may also contain carbohydrates, lignin, phenolic compounds, starch, etc., and it may be subjected to further separation steps or it may be subjected to fermentation.

The DES may conveniently be removed from the washing liquid and recycled if desired.

According to an embodiment, in the second step water is added to the biomass mixture, which is then agitated and subjected to separation.

The amount of added water is 1-8, preferably 1-4 times the times the volume of the DES.

This amount of water also contains the amounts of water possibly present in the first step or originating from the first step.

The temperature of the added water is 0-100°C, preferably 20-80°C, particularly preferably 40-75°C.

### Third step

In the third step proteins are separated using technique based on molecular size from the liquid fraction to yield a protein solution.
Proteins are isolated and enriched from the liquid fraction, which comprises the DES, low molecular weight compounds and water in addition the proteins.

The technique based on molecular size is selected from ultrafiltration, dialysis, membrane filtration methods and combinations thereof, whereby a protein solution is obtained.

Alternatively, technique based on isoelectric precipitation to yield a protein precipitate may be used. The protein is precipitated from the protein solution, either in the presence of or after removal of DES components, by isoelectric precipitation, in which the pH is adjusted to the proteins' isoelectric point causing the protein to precipitate.

The obtained protein solution may be further purified if desired by methods known in the art, concentrated, washed, and if necessary, enzyme treatments may be applied to depolymerize co-extracted polysaccharides to small saccharides, which can be easily removed by e.g. membrane technology.

The protein solution may be subjected to drying to obtain a dry protein extract.

The protein solution may be dried by freeze-drying, spray-drying, pressing, evaporating and by combinations thereof.

The obtained proteins may be used in human foods or nutritional supplements where they can serve as nutrients or performance proteins, in animal feeds, in chemical applications, and as specialty proteins.

It was surprisingly found that the selected DESs, particularly NaAcO:urea, were excellent and efficient solvents for solubilizing proteins, providing high yields of proteins

The process is fast, it can be reduced even to 2 h without significant yield losses.

Applying efficient washing protocols in the second step protein yields are increased, as washing the solid residue anew releases more protein from it.

In differential scanning calorimetry (DSC) experiments with different highly pure commercial proteins in aqueous DES solutions (0-60 wt% NaAcO:urea, 1:2), it was noticed that the protein denaturation temperature increased with the DES concentration in aqueous DES solutions, indicating that the protein potentially can be extracted in a more intact and natural form by DES extraction. This stabilization of proteins results in improved protein quality.

With the process typically less sugars and other non-protein compounds are co-extracted with the proteins, compared to the NaOH extraction method, and the proteins yields are higher.

The non-solubilized fraction can easily be hydrolyzed to fermentable sugars, whereby it is a very suitable feedstock for saccharification for the further production of biofuels and biochemicals via the sugar platform pathway, thus eliminating the generation of any considerable waste fractions in this process.

The DESs also selectively dissolve biopolymers, and in part open-up the matrix of the biomass material to allow for a higher degree of protein extraction by exposing more protein for easy dissolution. In addition, NaAcO: Urea (1:2) with 10 wt% water was noticed to have a high selectivity for dissolving protein vs carbohydrates from BSG (BSG extract contained 1 part carbohydrates for every 10 parts of protein).

The selected DESs are cheap solvents and they may be of food grade and easily recyclable.

The process of the disclosure can be used for producing proteins and protein fractions, which can further be used for the manufacture of protein products both for human nutrition and for animal feeds, as well as for other applications, such as specialty protein products in a simple, economic and efficient way.
A wide range of low cost feedstock, particularly agro-based side streams available in vast amounts can now be used for the manufacture of valuable protein products.

The invention is now illustrated in the following examples.

### Examples

### Example 1. Extraction of proteins from brewer's spent grain (BSG), rapeseed press cake (RSPC), and wheat bran (WB) using DES comprising sodium acetate and urea

Extraction experiments were carried out using a glass reactor equipped with mixer. Brewer's spent grain (BSG), rapeseed press cake (RSPC), and wheat bran (WB) were Wiley milled and treated with a DES comprising 90 % aqueous NaAcO:urea, (molar ratio 1:2). The DES solutions were prepared using dry salts, which were mixed with addition of water and heated up to the reaction temperature. The load of protein-containing starting material was 10 wt % in DES solution. The inside temperature in the vessel was 80°C, and the stirring speed of centrifugal stirrer paddles was 150 rpm and reaction time was adjusted to 16 hours. When the mixing was completed, the DES solution was filtered through 150 micrometer sieve from the solid residue, which was washed with hot water (8 times the amount of DES) to remove all free protein. The yield of extracted protein as compared to the total amount of protein in the starting material was 38.4 % from wheat bran, 44.4 % from RSPC and 37.5 % from BSG.

### Example 2. Extraction of proteins from brewer's spent grain (BSG) using DES comprising sodium acetate and urea

Extraction experiments were carried out using a glass reactor equipped with mixer. Brewer's spent grain (BSG) was Wiley milled and treated with a DES comprising 90 % aqueous NaAcO:urea, (molar ratio 1:2). The DES solution was prepared using dry salts, which were mixed with addition of 10 % water and heated up to the reaction temperature. The load of BSG was 5 wt % in DES solution. The inside temperature in the vessel was 80°C, and the stirring speed of centrifugal stirrer paddles was 150 rpm and the reaction time was adjusted to 1, 2 or 4 hours. When the mixing was completed, hot water (4 times the amount of DES) (80°C) was added to the DES solution, the solution was stirred with a magnetic stirrer and filtered through 150 micro meter sieve from the solid residue, which was further washed with an additional 200 ml of hot water (4 times the amount of DES) to remove all free protein. The protein extraction yields are presented in Figure 2. The reaction time could be reduced to 2 h without significant yield losses. Applying more efficient washing protocols after the initial extraction increases total protein extraction yields, as washing the solid residue anew in many cases released more protein from it. Extraction with aqueous NaOH solution (pH 11) at 30°C and 5 wt % BSG loading is provided as reference.

### Example 3. Separation and purification of proteins from aqueous DES solution

200 ml of extract obtained from the treatment of BSG in 90 % aqueous sodium acetate:urea (molar ratio 1:2), with general extraction parameters as described in examples 1 and 2 and 10 wt% extraction consistency and 20 h extraction time, was dialyzed using a dialysis membrane of regenerated cellulose with a molecular weight cutoff of 3 500 g/mol. The advance of the dialysis was followed by conductivity measurement, and the dialysis was deemed completed when the conductivity was below 2 µS/cm. The dialyzed liquid was further freeze-dried to yield light brown solids of a fibrous texture. The final protein content of the solid dry product was analyzed to 45 wt% and the protein yield of the purification phase was 63.4 %, calculated on basis that the extract taken for dialysis had an analyzed protein content of 1.77 g/L.

### Example 4. Extraction of proteins from brewer's spent grain (BSG) using DES comprising potassium acetate and urea

Extraction experiments were carried out using a glass reactor equipped with mixer. Brewer's spent grain (BSG) was Wiley milled and treated with a DES comprising 90 % aqueous KAcO:urea, (molar ratio 1:2). The DES solution was prepared using dry salts, which were mixed with addition of 10 % water and heated up to the extraction temperature. The load of BSG was 10 wt % in DES solution. The inside temperature in the vessel was 80oC, and the stirring speed of centrifugal stirrer paddles was 150 rpm and the reaction time was 4 hours. When the mixing was completed, hot water was mixed with the extraction mixture and the solution was stirred with a magnetic stirrer and filtered through 150 micrometer sieve from the solid residue, which was further washed with additional hot water to remove all free protein. The protein extraction yield was 65.6 %.

### Example 5. Extraction of proteins from brewer's spent grain (BSG) using DES comprising sodium formiate and urea

Extraction experiments were carried out using a glass reactor equipped with mixer. Brewer's spent grain (BSG) was Wiley milled and treated with a DES comprising 90 % aqueous Na-formiate:urea, (molar ratio 1:2). The DES solution was prepared using dry salts, which were mixed with addition of 10 % water and heated up to the extraction temperature. The load of BSG was 10 wt % in DES solution. The inside temperature in the vessel was 80°C, and the stirring speed of centrifugal stirrer paddles was 150 rpm and the reaction time was 4 hours. When the mixing was completed, hot water was mixed with the extraction mixture and the solution was stirred with a magnetic stirrer and filtered through 150 micrometer sieve from the solid residue, which was further washed with additional hot water to remove all free protein. The protein extraction yield was 63.8 %.

## Claims

1. A process for separating proteins and/or protein fractions from biomass materials, **characterized in that** the process comprises the steps, where in the first step biomass material containing streams and materials of plant or animal or algal or microbial origin, which contain proteins, or combinations thereof is mixed from 30 min to 30 hours at the temperature from 0°C to 100°C at the pressure 100 mbar - 30 bar with an aqueous deep eutectic solvent (DES) comprising a hydrogen bond acceptor selected from alkali metal carboxylates and earth alkaline metal carboxylates having the structure I:
[X]^{m} Yₙ^{o}, [I]
where
X denotes an alkali metal or earth alkaline metal selected from Na, K, Li, Be, Mg or Ca, preferably Na or K;
Y denotes a mono- or polyvalent carboxylate, which may carry a straight alkane chain with a length between 1 and 20 carbons, an aromatic moiety, a branched alkane, a multivalent carboxylic acid, alcohol groups, alkenes and alkynes with chain lengths of 1 to 20 carbon units;
m denotes the charge of the alkali or earth alkaline metal ion, +1 or +2;
n denotes the number of carboxylate anions, 1 to 2; and
o denotes the overall charge of the carboxylate anion, -1 or -2,
and urea as hydrogen bond donor, to obtain biomass mixture containing 1 - 40 wt% of the biomass material, calculated based on dry weight,
wherein deep eutectic solvent (DES) comprises a molar ratio of the alkali metal carboxylate or earth alkaline metal carboxylate to urea from 5:1 to 1:5 and the deep eutectic solvent (DES) comprises 0.01 - 4 mol of water with respect to the molar amount of the alkali metal carboxylate or earth alkaline metal carboxylate;
in the second step the biomass mixture is separated into a non-solubilized fraction and a liquid fraction, wherein 0 - 100°C water 1 - 8 times the volume of the deep eutectic solvent (DES) is added to the biomass mixture, the amount of water also containing the amounts of water possibly present in the first step or originating from the first step; and
in the third step proteins are separated from the liquid fraction using technique based on molecular size selected from ultrafiltration, dialysis, membrane filtration methods and combinations thereof to yield a protein solution, or using technique based on isoelectric precipitation to yield a protein precipitate.

2. The process according to claim 1, **characterized in that** the biomass material is from waste streams and side streams comprising plant material, animal material or algal material, and combinations thereof.

3. The process according to claim 1 or 2, **characterized in that** the carboxylate is selected from monovalent carboxylic acids, which carry alkane, alkene or alkyne chains of carbon chain length 1 - 20, in linear or branched configurations, polyvalent carboxylic acids, which carry alkane, alkene or alkyne chains of carbon chain length 1 - 20, in linear or branched configurations and optionally the carboxylate contains aromatic moieties or alcohol groups, preferably the carboxylate is selected from formiate, acetate, oxalate and propionate.

4. The process according to any one of claims 1 - 3, **characterized in that** the deep eutectic solvent (DES) comprises NaAcO and urea.

5. The process according to any one of claims 1 - 4, **characterized in that** temperature in the first step is from 20°C to 80°C.

6. The process according to any one of claims 1 - 5, **characterized in that** the biomass mixture contains 3-25 wt% of the biomass, calculated based on dry weight.

7. The process according to any one of claims 1 - 6, **characterized in that** in the first step the pressure is 1-20 bar.

8. The process according to any one of claims 1 - 7, **characterized in that** in the second step the separation is carried out at 40 - 100°C, preferably 60 - 85°C.

9. The process according to any one of claims 1 - 8, **characterized in that** in the second step the non-solubilized fraction is washed with water.

10. The process according to any one of claims 1 - 9, **characterized in that** the protein solution is purified.

11. The process according to any one of claims 1 - 10, **characterized in that** the protein solution is dried.

## Patentansprüche

1. Verfahren zur Abtrennung von Proteinen und/oder Proteinfraktionen aus Biomassematerialien, **gekennzeichnet dadurch, dass** das Verfahren die Schritte umfasst, in denen
im ersten Schritt ein Biomassenmaterial, das Ströme und Materialien pflanzlichen oder tierischen oder algalen oder mikrobiellen Ursprungs, welche Proteine enthalten, oder Kombinationen derselben enthält, 30 min bis 30 Stunden lang bei einer Temperatur von 0 °C bis 100 °C bei einem Druck von 100 mbar bis 30 bar mit einem wässrigen stark eutektischen Lösungsmittel (DES) gemischt wird, das einen Wasserstoffbrückenakzeptor umfasst, der ausgewählt ist aus Alkalimetallcarboxylaten und Erdalkalimetallcarboxylaten mit der Struktur I:
[X]^{m} Yₙ^{o}, [I]
wobei gilt:
X bezeichnet ein Alkalimetall oder Erdalkalimetall, ausgewählt aus Na, K, Li, Be, Mg oder Ca, bevorzugt Na oder K;
Y bezeichnet ein ein- oder mehrwertiges Carboxylat, das eine gerade Alkankette mit einer Länge zwischen 1 und 20 Kohlenstoffatomen, eine aromatische Einheit, ein verzweigtes Alkan, eine mehrwertige Carbonsäure, Alkoholgruppen, Alkene und Alkine mit Kettenlängen von 1 bis 20 Kohlenstoffeinheiten tragen kann;
m bezeichnet die Ladung des Alkali- oder Erdalkalimetallions, +1 oder +2;
n bezeichnet die Anzahl der Carboxylat-Anionen, 1 bis 2; und
o bezeichnet die Gesamtladung des Carboxylat-Anions, -1 oder -2,
und Harnstoff als Wasserstoffbrückendonator, um ein Biomassegemisch zu erzeugen, das 1 bis 40 Gew.-% des Biomassematerials enthält, bezogen auf Trockengewicht berechnet,
wobei das starke eutektische Lösungsmittel (DES) ein Molverhältnis des Alkalimetallcarboxylats oder Erdalkalimetallcarboxylats zu Harnstoff von 5:1 bis 1:5 umfasst und das starke eutektische Lösungsmittel (DES) 0,01 bis 0,4 Mol Wasser in Bezug auf die Molmenge des Alkalimetallcarboxylats oder Erdalkalimetallcarboxylats aufweist;
im zweiten Schritt das Biomassegemisch in eine nicht-solubilisierte Fraktion und eine flüssige Fraktion getrennt wird, wobei 0 bis 100 °C warmes Wasser dem Biomassegemisch in einem ein- bis achtfachen Volumen des starken eutektischen Lösungsmittels (DES) zugegeben wird, wobei die Wassermenge auch die Wassermengen mit enthält, die gegebenenfalls im ersten Schritt vorhanden sind oder vom ersten Schritt herrühren; und
im dritten Schritt Proteine mit Hilfe einer auf der Molekülgröße basierenden Technik, die aus Ultrafiltrations-, Dialyse-, Membranfiltrationsverfahren und deren Kombinationen ausgewählt ist, zur Herstellung einer Proteinlösung, oder mit Hilfe einer auf isoelektrischer Fällung basierenden Technik zur Herstellung eines Proteinniederschlags, aus der flüssigen Fraktion abgetrennt werden.

2. Verfahren nach Anspruch 1, **gekennzeichnet dadurch, dass** das Biomassematerial aus Abfallströmen und Seitenströmen stammt, die Pflanzenmaterial, Tiermaterial oder Algenmaterial und Kombinationen derselben umfassen.

3. Verfahren nach Anspruch 1 oder 2, **gekennzeichnet dadurch, dass** das Carboxylat aus einwertigen Carbonsäuren, die Alkan-, Alken- oder Alkinketten mit einer Kohenstoffkettenlänge von 1 bis 20 in linearen oder verzweigten Konfigurationen tragen, mehrwertigen Carbonsäuren, die Alkan-, Alken- oder Alkinketten mit einer Kohenstoffkettenlänge von 1 bis 20 in linearen oder verzweigten Konfigurationen tragen, ausgewählt ist und das Carboxylat optional aromatische Einheiten oder Alkoholgruppen enthält, wobei das Carboxylat vorzugsweise aus Formiat, Acetat, Oxalat und Propionat ausgewählt ist.

4. Verfahren nach einem der Ansprüche 1 bis 3, **gekennzeichnet dadurch, dass** das starke eutektische Lösungsmittel (DES) NaAcO und Harnstoff umfasst.

5. Verfahren nach einem der Ansprüche 1 bis 4, **gekennzeichnet dadurch, dass** die Temperatur im ersten Schritt 20 °C bis 80 °C beträgt.

6. Verfahren nach einem der Ansprüche 1 bis 5, **gekennzeichnet dadurch, dass** das Biomassegemisch 3 bis 25 Gew.-% der Biomasse enthält, bezogen auf Trockengewicht berechnet.

7. Verfahren nach einem der Ansprüche 1 bis 6, **gekennzeichnet dadurch, dass** im ersten Schritt der Druck 1 bis 20 bar beträgt.

8. Verfahren nach einem der Ansprüche 1 bis 7, **gekennzeichnet dadurch, dass** im zweiten Schritt die Abtrennung bei 40-100 °C, bevorzugt 60-85 °C, durchgeführt wird.

9. Verfahren nach einem der Ansprüche 1 bis 8, **gekennzeichnet dadurch, dass** im zweiten Schritt die nicht-solubilisierte Fraktion mit Wasser gewaschen wird.

10. Verfahren nach einem der Ansprüche 1 bis 9, **gekennzeichnet dadurch, dass** die Proteinlösung gereinigt wird.

11. Verfahren nach einem der Ansprüche 1 bis 10, **gekennzeichnet dadurch, dass** die Proteinlösung getrocknet wird.

## Revendications

1. Procédé pour séparer des protéines et/ou des fractions protéiques d'un matériau de biomasse, **caractérisé en ce que** ledit procédé comprend les étapes où dans la première étape, un matériau de biomasse contenant des flux et matériaux d'origine végétale ou animale ou algale ou microbienne, qui contiennent des protéines, ou des combinaisons de ceux-ci est mélangé de 30 min à 30 heures à une température de 0 °C à 100 °C à une pression de 100 mbar à 30 bars avec un solvant eutectique profond (DES) aqueux comprenant un accepteur de liaison hydrogène choisi parmi les carboxylates de métaux alcalins et les carboxylates de métaux alcalinoterreux présentant la structure I :
[X]^{m} Yₙ^{o}, [I]
où
X dénote un métal alcalin ou métal alcalinoterreux choisi parmi Na, K, Li, Be, Mg ou Ca, préférablement Na ou K ;
Y dénote un carboxylate monovalent ou polyvalent qui peut porter une chaîne droite d'alcane avec une longueur comprise entre 1 et 20 carbones, un groupement aromatique, un alcane ramifié, un acide carboxylique polyvalent, des groupes alcools, des alcènes et des alcynes avec des longueurs de chaîne de 1 à 20 unités de carbone ;
m dénote la charge de l'ion de métal alcalin ou alcalinoterreux, +1 ou +2 ;
n dénote le nombre d'anions de carboxylate, 1 à 2 ; et
o dénote la charge totale de l'anion de carboxylate, -1 ou -2,
et l'urée en tant que donneur de liaison hydrogène, pour obtenir un mélange de biomasse contenant 1 à 40 % en poids du matériau de biomasse, calculé par rapport au poids sec,
ledit solvant eutectique profond (DES) comprenant un rapport molaire entre le carboxylate de métal alcalin ou le carboxylate de métal alcalinoterreux et l'urée de 5:1 à 1:5 et ledit solvant eutectique profond (DES) comprenant 0,01 à 4 mois d'eau par rapport à la quantité molaire du carboxylate de métal alcalin ou du carboxylate de métal alcalinoterreux ;
dans la deuxième étape, le mélange de biomasse est séparé en une fraction non solubilisée et une fraction liquide, ledit mélange de biomasse étant additionné d'eau de 0 à 100 °C d'un volume 1 à 8 fois celui du solvant eutectique profond (DES), ladite quantité d'eau contenant également les quantités d'eau éventuellement présentes dans la première étape ou originaires de la première étape ; et
dans la troisième étape, des protéines sont séparées de la fraction liquide à l'aide d'une technique basée sur la taille moléculaire choisie parmi les procédés d'ultrafiltration, de dialyse, de filtration à membrane et leurs combinaisons pour produire une solution protéique, ou à l'aide d'une technique basée sur la précipitation isoélectrique pour produire un précipité protéique.

2. Procédé selon la revendication 1, **caractérisé en ce que** le matériau de biomasse provient de flux de déchets ou de flux latéraux comprenant du matériau végétal, matériau animal ou matériau algal, et des combinaisons de ceux-ci.

3. Procédé selon la revendication 1 ou 2, **caractérisé en ce que** le carboxylate est choisi parmi les acides carboxyliques monovalents qui portent des chaînes d'alcane, d'alcène ou d'alcyne avec une longueur de chaîne de carbone de 1 à 20 dans des configurations linéaires ou ramifiées, les acides carboxyliques polyvalents qui portent des chaînes d'alcane, d'alcène ou d'alcyne avec une longueur de chaîne de carbone de 1 à 20 dans des configurations linéaires ou ramifiées, et, en option, le carboxylate contient des groupements aromatiques ou groupes alcools, préférablement le carboxylate est choisi parmi le formiate, l'acétate, l'oxalate et le propionate.

4. Procédé selon l'une des revendications 1 à 3, **caractérisé en ce que** le solvant eutectique profond (DES) comprend le NaAcO et l'urée.

5. Procédé selon l'une des revendications 1 à 4, **caractérisé en ce que** la température dans la première étape est de 20 °C à 80 °C.

6. Procédé selon l'une des revendications 1 à 5, **caractérisé en ce que** le mélange de biomasse contient 3 à 25 % en poids de biomasse, calculé par rapport au poids sec.

7. Procédé selon l'une des revendications 1 à 6, **caractérisé en ce que** la pression dans la première étape est de 1 à 20 bars.

8. Procédé selon l'une des revendications 1 à 7, **caractérisé en ce que**, dans la deuxième étape, la séparation est réalisée à 40-100 °C, préférablement 60-85 °C.

9. Procédé selon l'une des revendications 1 à 8, **caractérisé en ce que**, dans la deuxième étape, la fraction non solubilisée est lavée avec de l'eau.

10. Procédé selon l'une des revendications 1 à 9, **caractérisé en ce que** la solution protéique est purifiée.

11. Procédé selon l'une des revendications 1 à 10, **caractérisé en ce que** la solution protéique est séchée.
